# EUROPÄISCHE PATENTANMELDUNG

(11) **EP 4 431 128 A2**
(43) Veröffentlichungstag der Anmeldung: **18.09.2024**
(21) Anmeldenummer: 24192175.8
(22) Anmeldetag: 10.08.2016
(51) Int. Cl.: A61M 1/28

(54) **PERITONEALDIALYSEGERÄT**

(30) Priorität: 11.08.2015 DE 102015010467
(62) Teilanmeldung aus: 20183083.3
(71) Anmelder: Fresenius Medical Care Deutschland GmbH, 61352 Bad Homburg (DE)
(72) Erfinder: WOLF, Klaus, 97450 Arnstein-Müdesheim (DE); HÖLZLE, Sebastian, 60320 Frankfurt (DE)
(74) Vertreter: Herrmann, Uwe

(57) **Zusammenfassung**

Die vorliegende Erfindung betrifft ein Peritonealdialysegerät mit einem Gerätegehäuse und mit einer Heizaufnahme sowie mit einer Mehrzahl von Lösungsbeuteln, wobei das Peritonealdialysegerät des Weiteren ein Schlauchset aufweist, das mit den Lösungsbeuteln in Verbindung steht, wobei die Mehrzahl von Lösungsbeuteln in der Heizschale angeordnet ist und wobei das Schlauchset keinen Heizbeutel aufweist, in den die von Lösungsbeuteln ablaufende Flüssigkeit hineinläuft.

## Beschreibung

Die vorliegende Erfindung betrifft ein Peritonealdialysegerät mit wenigstens einem Gerätegehäuse und mit wenigstens einer Heizschale sowie mit einer Mehrzahl von Lösungsbeuteln, wobei das Peritonealdialysegerät des Weiteren wenigstens ein Schlauchset aufweist, das mit den Lösungsbeuteln in Verbindung steht. Bei dem erfindungsgemäßen Gerät handelt es sich vorzugsweise um ein gravimetrisch arbeitendes Peritonealdialysegerät, welches ohne die Verwendung von Pumpen Dialysat von und zum Patienten fördert.

Aus dem Stand der Technik bekannte Peritonealdialysegeräte weisen ein Gerätegehäuse und eine sich oberhalb des Gerätegehäuses befindliche Infusionsstange auf, an der eine Mehrzahl von Lösungsbeuteln eingehängt sind, die eine dem Patienten zu verabreichende Dialyselösung enthalten. Des Weiteren ist eine Heizschale vorgesehen, in der sich ein Heizbeutel befindet. In den Heizbeutel wird die Dialyselösung aus den Lösungsbeuteln überführt und anschließend dem Patienten verabreicht.

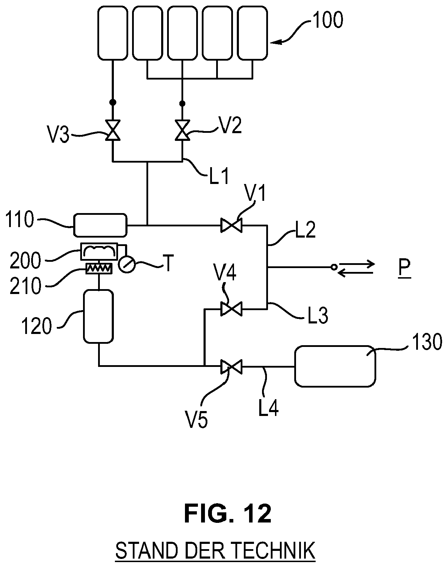

Ein schematischer Aufbau eines derartigen bekannten Gerätes ist aus Figur 12 ersichtlich.

In Figur 12 sind mit dem Bezugszeichen 100 die Lösungsbeutel gekennzeichnet. Das Bezugszeichen 110 kennzeichnet den Heizbeutel, der auf der Wäge- und Heizplatte 200 des Peritonealdialysegerätes liegt.

Das in den Heizbeutel 110 eingeführte frische Dialysat wird gewogen und temperiert und anschließend dem Patienten P zugeführt. Die Messung der Temperatur des Dialysats erfolgt mittels des Temperaturfühlers T. Zwischen den Lösungsbeuteln 100 und dem Heizbeutel 110 befinden sich die geräteseitig angeordneten Ventile V2 und V3, die mit dem Leitungsabschnitt L1 zusammenwirken. Mit dem Leitungsabschnitt L2 von dem Heizbeutel 110 zu dem Patienten P wirkt das geräteseitig angeordnete Einlaufventil V1 zusammen, das den Fluidfluss von dem Heizbeutel 110 zu dem Patienten P steuert.

Wird das verbrauchte Dialysat mittels des Leitungsabschnittes L3 von dem Patienten abgeführt, gelangt dieses zunächst in den Wägebeutel 120. Der Wägebeutel steht mit einer Wägezelle 210 in Verbindung, die das Gewicht des Wägebeutels 120 ermittelt. Mit dem Leitungsabschnitt L3 wirkt das geräteseitig angeordnete Auslaufventil V4 zusammen.

Nach dem Wiegen des verbrauchten Dialysats durch die Wägezelle 210 gelangt das Dialysat durch den Leitungsabschnitt in den Abfallbeutel 130, mit dem das verbrauchte Dialysat verworfen wird. Mit dem Leitungsabschnitt L4 wirkt das geräteseitig angeordnete Drainageventil V5 zusammen, das parallel zu dem Auslaufventil V4 angeordnet ist.

Die Leitungsabschnitte L1 bis L4 bilden das Schlauchset.

Nachteilig an dem aus dem Stand der Technik bekannten Gerät ist es, dass zur Positionierung der Lösungsbeutel eine große Höhe (ca. 1,7 m) überwunden werden muss und somit die Beladung des Gerätes entsprechend erschwert ist. Abgesehen davon ist die Stabilität aufgrund des vergleichsweise hohen Schwerpunktes des Gerätes durch die Bestückung der Beutel (i.d.R. 10 l (2 x 5 l) oder 30 l (5 x 6 l)) sowie durch den auf der Heizschale 110 liegenden Beutel eingeschränkt.

Des Weiteren sind aufgrund der großen Entfernung zwischen den Lösungsbeuteln und der Heizschale entsprechend lange Schlauchleitungen des Schlauchsets erforderlich.

Der vorliegenden Erfindung liegt die Aufgabe zugrunde, ein Peritonealdialysegerät der eingangs genannten Art dahingehend weiterzubilden, dass dieses einen kompakten Aufbau aufweist.

Diese Aufgabe wird durch ein Peritonealdialysegerät mit den Merkmalen des Anspruchs 1 gelöst. Danach ist vorgesehen, dass die Mehrzahl von Lösungsbeuteln in der wenigstens einen Heizschale angeordnet ist und dass das Schlauchset mit keinem Heizbeutel in Verbindung steht, in den die von Lösungsbeuteln ablaufende Flüssigkeit hineinläuft. Abweichend von der aus dem Stand der Technik bekannten Anordnung befindet sich wenigstens einer, vorzugsweise alle Lösungsbeutel, die das frische Dialysat oder eine Flüssigkeit zur Herstellung des Dialysats enthalten, nicht an einer Infusionsstange, sondern in der Heizschale des Gerätes. Es werden somit nicht zunächst die Lösungsbeutel an einem Infusionsständer bzw. an der Infusionsstange aufgehängt, die sich ausgehend von dem Gerätegehäuse nach oben erstreckt. Stattdessen werden die Lösungsbeutel gemeinsam in eine oder mehrere Heizschalen eingelegt. Eine Infusionsstange ist somit vorzugsweise nicht vorhanden.

Gleichwohl ist von der Erfindung auch eine Ausführungsform umfasst, die eine Infusionsstange aufweist, an der sich ein oder mehrere Lösungsbeutel befinden.

Vorzugweise befindet sich die Heizschale oberhalb des Gerätegehäuses des Peritonealdialysegerätes, in dem sich die Gerätesteuerung und Anzeige- und/oder Bedienelemente befinden. Die Heizschale kann direkt mit dem Gerätegehäuse verbunden sein.

Die Heizschale weist eine Größe auf, so dass in dieser möglichst alle für eine Behandlung erforderlichen Lösungsbeutel aufgenommen werden können. Von der Erfindung ist jedoch auch der Fall umfasst, dass nicht alle für eine Behandlung erforderlichen Lösungsbeutel in der Heizschale angeordnet sind, sondern nur ein Teil von diesen.

Denkbar ist es, dass die Lösungsbeutel in der Heizschale nebeneinander und/oder übereinander, d.h. gestapelt angeordnet sind. Durch den direkten Kontakt der übereinander gestapelten Lösungsbeutel stellt sich ein thermisches Gleichgewicht ein, aufgrund dessen auch von der Heizplatte weiter entfernt angeordnete Lösungsbeutel ausreichend aufgeheizt werden.

Bevorzugt ist es, wenn die Lösungsbeutel in der Heizschale nebeneinander angeordnet sind.

Der Vorteil der erfindungsgemäßen Anordnung der Lösungsbeutel besteht in einer geringeren Bauhöhe des Gesamtgerätes, da in einer bevorzugten Ausgestaltung der Erfindung auf eine Infusionsstange verzichtet werden kann und die Lösungsbeutel nahe des Gerätegehäuses angeordnet sind.

Ein weiterer Vorteil besteht in einer vereinfachten Handhabung, da die Lösungsbeutel nur in die Heizschale gelegt werden müssen, die sich vorzugsweise in geringerer Höhe befindet, als die Einhängepositionen einer Infusionsstange bei bekannten Geräten. Daraus ergibt sich der weitere Vorteil, dass die Schlauchleitungen in der Länge reduziert werden können.

Eine bevorzugte Länge des Schlauchsets liegt im Bereich von 3 m bis 4,5 m, vorzugsweise im Bereich von 3,5 m bis 4,0 m.

Ein weiterer Vorteil besteht darin, dass kein separater Heizbeutel erforderlich ist, in den die aus dem Lösungsbeutel stammende Lösung bzw. das Dialysat hineinläuft. Der Verzicht wird dadurch möglich, dass die Lösungsbeutel und somit das darin befindliche Dialysat selbst beheizt werden.

Das Schlauchset von den Lösungsbeuteln zum Patienten weist somit keinen Heizbeutel auf und ist aus diesem Grund sowie aufgrund der geringeren notwendigen Schlauchlänge vereinfacht.

Die vorliegende Erfindung betrifft des Weiteren ein Peritonealdialysegerät mit wenigstens einer Wägeeinrichtung, an der zumindest ein Aufnahmebeutel zur Aufnahme des ablaufenden Dialysators angeordnet ist, sowie mit wenigstens einem Schlauchset, das mit dem oder den Aufnahmebeuteln in Verbindung steht, wobei der wenigstens eine Aufnahmebeutel nicht mit einem Abfallbeutel, in dem das abgelaufene Dialysat gesammelt wird, in Verbindung steht, sondern selbst den Abfallbeutel und den Wägebeutel, d.h. den mittels der Wägeeinrichtung gewogenen Beutel bildet.

Bekannte gravimetrische Peritonealdialysegeräte verfügen zur Bilanzierung über einen separaten Wägebeutel, der in Figur 12 mit dem Bezugszeichen 120 gekennzeichnet ist. Bei bekannten Peritonealdialysegeräten wird das verbrauchte Dialysat, d.h. das Drainagefluid sodann in einen Drainbehälter abgelassen (Bezugszeichen 130 in Figur 12).

Gemäß dem in Anspruch 2 geschützten Aspekt der Erfindung wird auf einen separaten Wägebeutel verzichtet, da das ablaufende, verbrauchte Dialysat direkt in einen Drainbehälter, d.h. in den Ablaufbeutel abgelassen wird. Daraus ergibt sich eine entsprechende Vereinfachung des Schlauchsets auf der Ablaufseite. Gemäß diesem Aspekt der Erfindung ist somit kein Beutel (Wägebeutel) vorgesehen, der nur die Aufgabe der Wägung des verbrauchten Dialysats hat und von dem aus das Dialysat in einen Abfallbeutel geleitet wird. Vielmehr dient der Ablaufbeutel als Wägebeutel und bildet auch gleichzeitig den Abfallbeutel. Ein weiterer Abfallbeutel, in den das verbrauchte Dialysat überführt wird, ist nicht vorgesehen.

Auch eine Kombination der beiden erfindungsgemäßen Gedanken gemäß der Ansprüche 1 und 2 ist denkbar und von der Erfindung mit umfasst.

Wie oben ausgeführt, ist es vorteilhaft, wenn das Peritonealdialysegerät keine Infusionsstange aufweist, an der Lösungsbeutel befestigt sind. Vorzugsweise sind alle Lösungsbeutel, die das frische Dialysat enthalten, in der Heizschale aufgenommen.

Gleichwohl ist von der Erfindung auch eine Ausführungsform umfasst, in der eine oder mehrere Infusionsstangen vorgesehen sind.

In einer bevorzugten Ausgestaltung der Erfindung weist das Peritonealdialysegerät wenigstens ein Gerätegehäuse auf, wobei die Heizschale oberhalb des Gerätegehäuses angeordnet ist und vorzugsweise unmittelbar mit dem Gerätegehäuse verbunden ist.

Werden alle für eine Behandlung benötigten Lösungsbeutel in der Heizschale aufgenommen, muss diese eine entsprechende Größe aufweisen.

Denkbar ist es, dass die Lösungsbeutel in der Heizschale übereinander und/oder vorzugsweise nebeneinander angeordnet sind. Bei einer Anordnung der Lösungsbeutel übereinander ergibt sich der Vorteil, dass aufgrund eines sich einstellenden thermischen Gleichgewichts auch nicht unmittelbar mit der Heizschale in Kontakt stehende Lösungsbeutel erwärmt werden.

Das wenigstens eine Schlauchset kann derart ausgebildet sein, dass es zeitgleich mit zwei oder mehr als zwei Lösungsbeuteln in Fluidverbindung steht, so dass eine Mischlösung hergestellt werden kann. Durch die Anordnung mehrerer und vorzugsweise aller für eine Behandlung benötigter Lösungsbeutel in bzw. auf der Heizschale können wenigstens zwei, vorzugsweise drei Lösungsbeutel gleichzeitig konnektiert werden und z.B. über ein Y-Stück mit dem Zuleitungsschlauch zum Patientenkonnektor der Patientenleitung verbunden werden. Dadurch lässt sich eine weitere Verkürzung des Schlauches erreichen.

Die konnektierten Lösungsbeutel werden vorzugsweise nebeneinander angeordnet, um eine gleichmäßige Entleerung der Lösungsbeutel zu erzielen.

Weisen die konnektierten Lösungsbeutel unterschiedliche Konzentrationen auf, kann bei gleichzeitiger Entnahme des Dialysats aus den Lösungsbeuteln ein gewünschtes Mischungsverhältnis in der Lösung eingestellt werden, die dem Patienten verabreicht wird.

Vorzugsweise kann aufgrund der Anordnung der Lösungsbeutel nicht nur der Heizbeutel entfallen, sondern auch das dazugehörige Ventil zur Flusssteuerung vom Heizbeutel zum Patienten, das in Figur 12 mit dem Bezugszeichen V1 gekennzeichnet ist.

Vorzugsweise wirkt das Schlauchset in dem Bereich zwischen einem Lösungsbeutel und dem Ende des Schlauchsets, mit dem dieses mit dem Patientenzugang verbunden wird, mit nur genau einem Ventil (Fluidsteuerungsventil) zusammen. Den mehreren Lösungsbeuteln ist abgesehen von dem Last bag somit insgesamt nur ein Ventil zugeordnet. Bei geöffnetem Ventil läuft Dialysat aus dem betreffenden Lösungsbeutel zum Patienten, bei geschlossenem Ventil unterbleibt der Einlauf. Zusätzlich kann ein Ventil für den sognannten "Last bag" vorgesehen sein, dass den Einlauf der darin befindlichen Lösung zum Patienten steuert.

Durch den Entfall des Heizbeutels ergibt sich abgesehen von einer Vereinfachung des Schlauchsets auch eine verbesserte Bilanzierung, da ein Nachlaufen von Dialysat aus dem Heizbeutel entfällt und auch keine Restflüssigkeit in dem Heizbeutel zurückbleibt.

Vorzugsweise weist die Wägeeinrichtung wenigstens eine Wägeschale auf, wobei der oder die Aufnahmebeutel in der Wägeschale angeordnet sind.

Aufgrund des Wegfalls eines Wägebeutels kann auch auf das zugehörige Ventil verzichtet werden, das in Figur 12 mit dem Bezugszeichen V5 gekennzeichnet ist.

Vorzugsweise ist vorgesehen, dass in dem Schlauchset zwischen dem Ende des Schlauchsets, mit dem dieses mit einem Patientenzugang in Verbindung steht und dem Aufnahmebeutel nur genau ein Ventil (Drainageventil) vorgesehen ist. Ist dieses geöffnet, läuft verbrauchtes Dialysat in den Aufnahmebeutel, ist es geschlossen, verhindert das Ventil ein Ablauf von Dialysat aus dem Patienten.

Der oder die Aufnahmebeutel ist vorzugsweise in der Wägeschale und vorzugsweise darin liegend aufgenommen.

Die Wägeschale kann über wenigstens eine Haltevorrichtung mit einer Wägezelle des Peritonealdialysegerätes verbunden sein. Die Haltevorrichtung erstreckt sich vorzugsweise in vertikaler Richtung. Diese Ausführung bringt den Vorteil mit sich, dass die Wägeschale und damit auch der Aufnahmebeutel, d.h. der Drainagebeutel in unmittelbarer Bodennähe angeordnet werden können, so dass der gravimetrische Bereich gut ausgenutzt werden kann bzw. eine hinreichende Höhendifferenz zwischen Patient und dem Aufnahmebeutel besteht. Bei bekannten Geräten ist ein Wägebeutel unterhalb des Gerätegehäuses, nicht jedoch in Bodennähe angeordnet, so dass sich eine entsprechend kleinere Höhendifferenz ergibt.

Bevorzugt ist es, wenn die Wägeschale in einer Höhe von < 20 cm, vorzugsweise < 10 cm über dem Boden angeordnet ist, auf dem das Peritonealdialysegerät steht.

Aufgrund des erreichten vergrößerten Abstandes zwischen Heizschale und Drainschale bzw. Wägeschale, d.h. der Schale, in der sich der wenigstens eine Aufnahmebeutel befindet, lässt sich eine optimale Flussgeschwindigkeit erzielen.

Geht man von einer Höhe des Patienten von ca. 50 bis 60 cm über dem Boden aus, entspricht die Einlaufgeschwindigkeit für das frische Dialysat der Auslaufgeschwindigkeit des verbrauchten Dialysats, wenn der Abstand zwischen der Heizschale und dem Ablaufbeutel ca. 1 m ± 20 cm beträgt und sich der Aufnahmebeutel bzw. die diesen aufnehmende Schale kurz über dem Boden befindet.

Weist der Aufnahmebeutel eine flache Form auf, kann weiterhin eine vom Füllgrad des Aufnahmebeutels unabhängige konstante Auslaufgeschwindigkeit realisiert werden.

Denkbar ist es, dass die Wägezelle in oder an dem Gerätegehäuse angeordnet ist und/oder dass die Haltevorrichtung eine Folie oder ein sonstiges flexibles Material umfasst, in dem die Schale eingelegt ist oder dass die Haltevorrichtung ein Gestänge umfasst, das sich vorzugsweise auf der von der Vorderseite des Gerätegehäuses abgewandten Rückseite des Gerätegehäuses befindet.

Die Wägeschale, d.h. die Schale, in der sich der oder die Aufnahmebeutel befinden, kann z.B. über eine beidseitig geführte, z.B. transparente Folie gehalten werden. Bevorzugt ist jedoch eine mechanische Haltevorrichtung mit einem Gestänge. Besonders vorteilhaft ist es, wenn ein ausbalanciertes Gestänge auf der hinteren Seite des Peritonealdialysegerätes bzw. von dessen Gerätegehäuse verwendet wird, wobei die Vorderseite frei ist von Befestigungsmitteln, so dass eine gute Zugänglichkeit zu der Schale bzw. zu dem darin aufgenommenen Aufnahmebeutel gegeben ist.

Diese Anordnung der Haltevorrichtung erleichtert das Einlegen des oder der Aufnahmebeutel in die Schale von vorne, d.h. von der Bedienseite des Peritonealdialysegerätes.

Vorzugsweise liegt der Abstand zwischen der Wägeschale und der Heizschale in Höhenrichtung im Bereich zwischen 80 cm und 1,2 m und vorzugsweise zwischen 90 cm und 1,1 m.

Weiterhin kann vorgesehen sein, dass das Peritonealdialysegerät zumindest ein Gerätegehäuse aufweist und dass sich das oder die Fluidsteuerungsventile, die zwischen dem Lösungsbeutel und dem Patienten angeordnet sind, an dem Gerätegehäuse des Peritonealdialysegerätes befindet.

Das wenigstens eine Drainageventil, das sich zwischen der Patientenleitung und dem Auslaufbeutel befindet, kann sich an einem Gerätegehäuseträger befinden, wobei vorzugsweise vorgesehen ist, dass sich das Drainageventil in einer Höhe von 30 cm bis 60 cm, vorzugsweise in einer Höhe von 35 cm bis 55 cm über den Boden befindet, auf dem das Peritonealdialysegerät steht. Dies erlaubt eine bequeme Bedienung des Ventils durch den Patienten, dabei ist eine umso höhere Anordnung umso besser. Gleichzeitig ist der hydrostatische Druck im System noch ausreichend hoch, um die im Schlauchsystem befindliche Luft in einem Spülschritt entfernen zu können, dabei ist eine umso niedrige Anordnung umso besser.

Bekannte Peritonealdialysegeräte weisen einen spinnenartigen Fuß mit mehreren sich von einem Zentrum nach außen erstreckenden Beinen auf, an denen Rollen angeordnet sind. Vorzugsweise ist vorgesehen, dass das Peritonealdialysegerät mit einem U-förmigen Standfuß ausgebildet ist. Dieser kann schmal ausgeführt sein. Er erlaubt eine verbesserte Standfestigkeit sowie Stabilität des Gerätes und ist abgesehen davon platzsparend. Sind die Schenkel des Fußes flach ausgeführt, erlaubt dies darüber hinaus die Anordnung der Schale für den Aufnahmebeutel in Bodennähe. Vorzugsweise ist der Standfuß ohne Rollen ausgeführt.

Das Peritonealdialysegerät weist vorzugsweise einen Abstand zwischen der Heizschale als obersten Punkt und der Schale für den Aufnahmebeutel als unteren Punkt von < 1,2 m und vorzugsweise von < 1,1 m auf. Das Gerät ist somit kompakt und stabil. Der Abstand liegt vorzugsweise zwischen 80 cm und 1,2 m. Diese Differenz ist erforderlich, um einen einwandfreien Einlauf und Auslauf zu gewährleisten.

Um die Mehrzahl von Lösungsbeuteln sicher halten zu können, weist die Heizschale und/oder die Wägeschale in bevorzugter Ausgestaltung einen Boden und sich von dem Boden nach oben erstreckende Seitenwandungen bzw. seitlich erhöhte Befestigungsplatten auf, wobei vorzugsweise vorgesehen ist, dass eine oder mehrere der Seitenwandungen bzw. der Befestigungsplatten von dem Boden abnehmbar oder relativ zu dem Boden z.B. über wenigstens ein Scharnier verschwenkbar sind. Dies vereinfacht das Einlegen und das Entnehmen von Beuteln.

Zur Abnahme und Montage der Seitenwandungen bzw. der Befestigungsplatten können Steckverbindungen vorgesehen sein.

Insgesamt ermöglicht das erfindungsgemäße Peritonealdialysegerät eine optimierte gravimetrische Peritonealdialysebehandlung.

In einer bevorzugten Ausgestaltung der Erfindung kann auf den Heizbeutel sowie auch auf den Wägebeutel verzichtet werden. Des Weiteren kann die Schlauchlänge zwischen den Lösungsbeuteln und dem Y-Stück zum Patientenanschluss (bei gleichzeitigem Anschluss mehrerer Lösungsbeutel) von 1,5 m auf weniger als 1 m verkürzt werden.

Denkbar ist es, dass das Schlauchset erste Leitungsabschnitte aufweist, die sich von den Anschlüssen für die Lösungsbeutel zu einem Y-Stück erstrecken, wobei mit dem Y-Stück ein zweiter Leitungsabschnitt in Verbindung steht, der sich von dem Y-Stück zu dem Anschluss für den oder die Aufnahmebeutel oder eine oder mehrere damit in Verbindung stehende Leitungen erstreckt, und wobei mit dem Y-Stück ein dritter Leitungsabschnitt in Verbindung steht, der sich von dem Y-Stück zu dem Anschluss für die Patientenleitung erstreckt, wobei die ersten Leitungsabschnitte jeweils eine Länge im Bereich von 50 cm bis 80 cm, vorzugsweise im Bereich von 60 cm bis 70 cm, wobei der zweite Leitungsabschnitt eine Länge im Bereich von 25 cm bis 45 cm, vorzugsweise im Bereich von 30 cm bis 40 cm und wobei der dritte Leitungsabschnitt eine Länge im Bereich von 1,9 m bis 2,2 m, vorzugsweise im Bereich von 2,0 m bis 2,1 m aufweisen. Anstelle eines Y-Stückes ist auch jeder andere Konnektor denkbar und von der Erfindung umfasst.

Eine weitere Vergünstigung des Schlauchsets besteht darin, dass ein Brechkonnektor verwendet wird, wie er aus der EP 1 351 726 bekannt ist, auf die insoweit Bezug genommen wird. So können nach der Behandlung die entleerten Lösungsbeutel durch Brechen des Konnektors vom Schlauchset abgetrennt und mit einem neuen Schlauch bzw. Schlauchset verbunden werden. Diese Beutel dienen dann als Auslaufbeutel, d.h. als Drainagebeutel.

Die vorliegende Erfindung betrifft des Weiteren die Verwendung eines Schlauchsets in einem erfindungsgemäßen Peritonealdialysegerät. Das Schlauchset steht nicht mit einem Heizbeutel und/oder nicht mit einem Wägebeutel in Verbindung, aus dem das verbrauchte Dialysat in einen Abfallbeutel überführt wird. Das Schlauchset kann gemäß der weiteren, oben genannten Merkmale ausgeführt sein.

Weitere Vorteile und Einzelheiten der Erfindung werden anhand eines in der Zeichnung dargestellten Ausführungsbeispiels näher erläutert.

Es zeigen:
- Figur 1:: eine perspektivische Ansicht eines Peritonealdialysegerätes gemäß der Erfindung,
- Figur 2:: eine schematische Ansicht eines Peritonealdialysegerätes gemäß der Erfindung,
- Figur 3:: eine weitere perspektivische Ansicht eines Peritonealdialysegerätes gemäß der Erfindung,
- Figur 4:: Ansichten einer Heizschale ohne und mit Lösungsbeuteln,
- Figur 5:: Ansichten einer Heizschale mit darin nebeneinander angeordneten Lösungsbeuteln,
- Figur 6:: eine Ansicht des Drainageventils und der Wägeschale,
- Figur 7, 8:: eine Ansicht des Schlauchsets ohne und mit Anschlussleitungen für die Aufnahmebeutel,
- Figur 9:: eine Darstellung der Schritte zum Trennen der Leitungen der Lösungsbeutel von einem gebrauchten Schlauchset und zum Verbinden der Anschlussleitungen für die Aufnahmebeutel mit einem neuen Schlauchset,
- Figur 10:: unterschiedliche Ausführungen von Schlauchsets,
- Figur 11:: ein Schlauchset mit zwei Leitungen für Lösungsbeutel, einer Anschlussleitung für den "Last bag", einer Leitung für die Verbindung mit der Patientenleitung und einer Leitung für die Verbindung mit einem Aufnahmebeutel oder mit einer oder mehreren zu den Aufnahmebeuteln führenden Leitungen,
- Figur 12:: eine schematische Ansicht der Komponenten eines Peritonealdialysegerätes gemäß dem Stand der Technik.

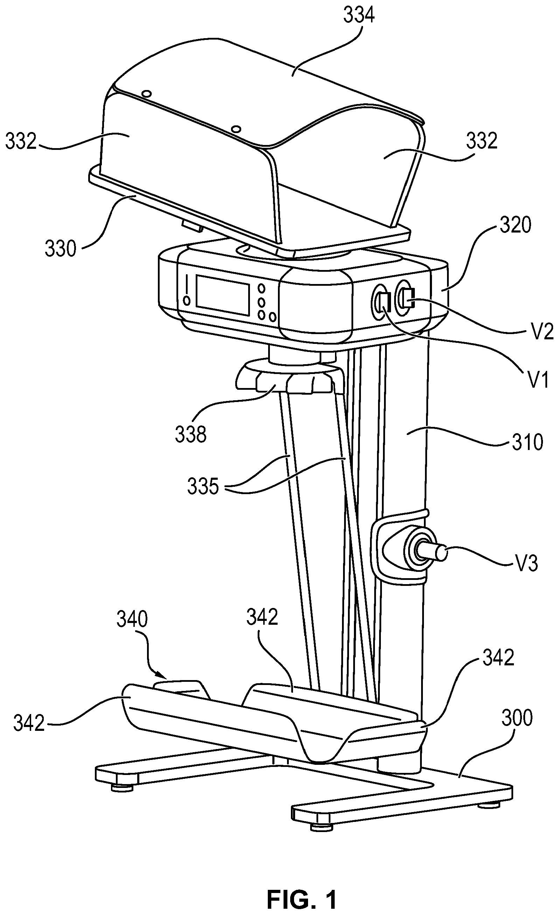

Figur 1 zeigt ein Peritonealdialysegerät gemäß der vorliegenden Erfindung in einer perspektivischen Ansicht.

Das Peritonealdialysegerät weist einen U-förmigen Standfuß 300 auf, von dem sich nach vertikal oben ein Gerätegehäuseträger 310 erstreckt. An dessen oberen Ende befindet sich das Gerätegehäuse 320. In dem Gerätegehäuse 320 befindet sich die zum Betrieb des Gerätes erforderliche Elektronik, wie z.B. Steuer- und Regelungseinheiten, sowie die Bedien- und/oder Anzeigeeinheiten.

Unmittelbar oberhalb des Gerätegehäuses 320 ist direkt mit diesem verbunden die Heizschale 330 angeordnet, die zur Aufnahme von Lösungsbeuteln dient, die frisches, dem Patienten zuzuführendes Dialysat enthalten.

Unten an dem Gerätegehäuse 320 bzw. an der Wägezelle 338 befindet sich ein Gestänge 335, an dem die Wägeschale 340 angeordnet ist. Die Wägeschale 340 dient zur Aufnahme eines oder mehrerer Aufnahmebeutel, in die das gebrauchte, vom Patienten stammende Dialysat gelangt.

Wie dies aus Figur 1 ersichtlich ist, befindet sich die Wägeschale 340 unmittelbar über dem Fußboden, auf dem das Peritonealdialysegerät mit seinem Standfuß 300 aufsteht. Aus Figur 1 geht weiter hervor, dass der Standfuß ein flaches Profil aufweist, so dass die Wägeschale 340 weit unten angeordnet werden kann.

Der Abstand zwischen dem Boden der Wägeschale 340 und dem Fußboden beträgt beispielsweise wenige cm, z.B. 5 cm bis 10 cm und vorzugsweise 7,5 cm bis 8,5 cm.

Der Abstand zwischen dem Boden der Heizschale 330 und dem Boden der Wägeschale 340 liegt vorzugsweise zwischen 80 cm und 1,2 m.

Die Fluidsteuerung zum und vom Patienten erfolgt über Ventile, wobei das oder die Ventile für die Fluidverbindung zwischen dem bzw. den Lösungsbeuteln, die sich in der Heizschale 330 befinden, und dem Patienten am Gerätgehäuse 320 angeordnet sind. Diese Ventile sind in Figur 1 mit dem Bezugszeichen V1, V2 gekennzeichnet. Das Drainageventil V3, das zwischen dem Patienten und dem Aufnahmebeutel für verbrauchtes Dialysat angeordnet ist, befindet sich an dem Gerätegehäuseträger 310. Es ist in einer Höhe von ca. 40 cm bis 50 cm und vorzugsweise von 45 cm über dem Boden angeordnet. Dies erlaubt eine bequeme Bedienung durch den Patienten.

Wie aus Figur 1 ersichtlich, erstreckt sich das Gestänge 335 von der Rückseite der Wägeschale 340 nach oben zu der Unterseite des Gerätegehäuses 320. Somit ist eine gute Zugänglichkeit von der Vorderseite zu der Wägeschale 340 gegeben.

Das Gestänge 335 ist an einer Wägezelle 338 angeordnet, die sich an oder in dem Gerätegehäuse 320 befindet.

Sowohl die Heizschale 330 als auch die Wägeschale 340 weisen eine Auflagefläche für den oder die Beutel auf, die den Boden darstellt. Ausgehend von dem Boden erstrecken sich Seitenwände 332 und 342 nach oben. Diese haben die Aufgabe, die jeweils aufgenommenen Beutel sicher in der Schale zu halten. Dies ist insbesondere dann von Bedeutung, wenn in der Heizschale 330 und in der Wägeschale 340 eine Mehrzahl von Beuteln aufgenommen ist.

Die Seitenwände 332 und 342 können durch eine Steckverbindung befestigt sein oder relativ zu der Auflagefläche verschwenkbar sein, so dass die Beutel leicht eingelegt und entnommen werden können.

Die Heizschale 330 ist darüber hinaus mit einer oberen Abdeckung 334 versehen, die die Aufgabe hat, die Wärme möglichst im Bereich der Lösungsbeutel zu halten, die sich in der Heizschale 330 befinden.

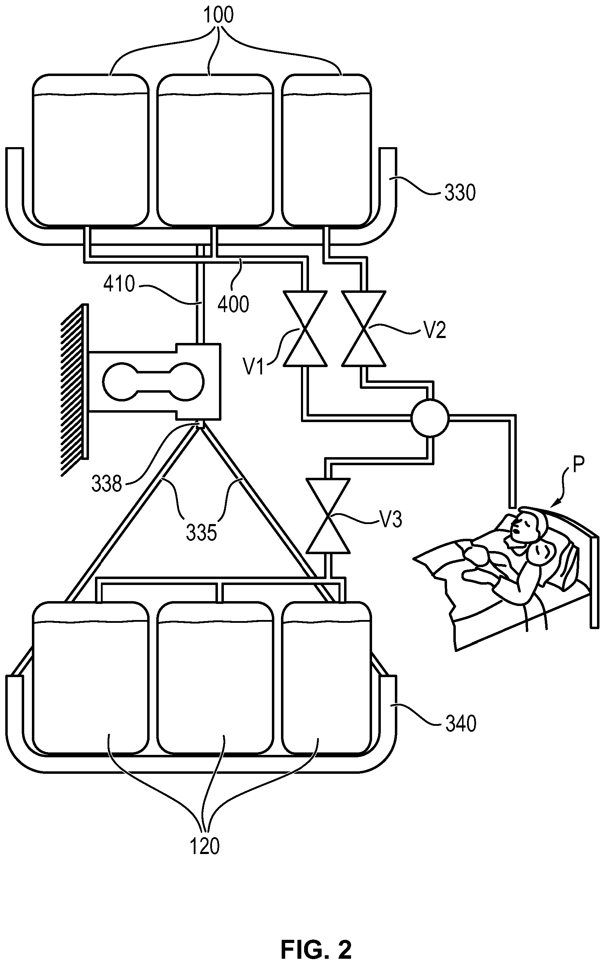

Figur 2 zeigt in schematischer Ansicht das Peritonealdialysegerät gemäß Figur 1. In dem hier gezeigten Ausführungsbeispiel befinden sich in der Heizschale 330 drei Lösungsbeutel 100, die jeweils Dialysat enthalten, das dem Patienten P zuzuführen ist. Die beiden links dargestellten Lösungsbeutel sind durch ein Y-Stück 400 miteinander verbunden. Stromabwärts des Y-Stücks 400 befindet sich das Ventil V1. Der rechts dargestellte Lösungsbeutel ist ebenfalls durch ein Ventil V2 von dem Patienten P getrennt.

Durch Öffnen der Ventile V1, V2 wird dem Patienten P das jeweilige Dialysat zugeführt.

Die Heizschale 330 ist mit einem Gestänge 410 oben auf dem Gerätegehäuse 320 fixiert. Es kann mit einer Wägeeinrichtung derart verbunden sein, dass das Gewicht der Lösungsbeutel 100 in der Heizschale 330 gemessen werden kann.

Unterhalb des Gerätegehäuses 320 befindet sich die Wägeschale 340, in der mehrere Aufnahmebeutel 120 zur Aufnahme des von dem Patienten kommenden, verbrauchten Dialysats angeordnet sind. Die Anzahl und das Volumen der Aufnahmebeutel 120 entsprechen dem der Lösungsbeutel 100.

Zwischen dem Patienten und dem Aufnahmebeuteln 120 befindet sich das Ventil V3. Wird dieses geöffnet, gelangt das Dialysat vom Patienten in die Aufnahmebeutel. In den Aufnahmebeuteln 120 wird das verbrauchte Dialysat gesammelt. Eine Weiterleitung zu einem oder mehreren anderen Beuteln erfolgt nicht.

Ein Vergleich der Anordnung gemäß Figur 1 und 12 zeigt, dass bei der erfindungsgemäßen Anordnung auf einen eigenen Heizbeutel verzichtet werden kann, da die Lösungsbeutel selbst in der Heizschale aufgenommen sind. Des Weiteren kann bei der erfindungsgemäßen Anordnung auf einen nur zum Wiegen dienenden Wägebeutel verzichtet werden, da die Aufnahmebeutel selbst gewogen werden.

Dadurch ist nicht nur ein Verzicht auf die Heiz- und Wägebeutel möglich, sondern auch ein Verzicht auf die Ventile V1 und V5.

Dies bringt den Vorteil eines entsprechend vereinfachten Schlauchsets mit sich. Aufgrund der geringeren Höhe des Gesamtgerätes können die verwendeten Schläuche gegenüber bekannten Anordnungen kürzer ausgeführt werden.

Wie oben ausgeführt, lässt sich eine weitere Vereinfachung dadurch erzielen, dass das Schlauchset mit wenigstens einem Brechkonnektor versehen wird, wie er aus der EP 1 351 726 bekannt ist. Die entleerten Lösungsbeutel 100 werden nach der Behandlung durch Brechen des Konnektors abgetrennt und mit einem neuen Schlauchset verbunden. Diese Beutel können dann in dem neuen Schlauchset als Drainagebeutel bzw. als Aufnahmebeutel dienen.

Weitere Vorteile bestehen darin, dass kein letzter Beutel mit verdünnter Lösung vorgesehen werden muss, und dass sich kürzere Behandlungszeiten erzielen lassen, da die wiederholte Füllung eines Heizbeutels und eine Wägebeutels entfällt.

Aufgrund der Tatsache, dass das Drainageventil relativ weit unten angeordnet wird, kann sich der Patient ebenfalls in einer geringeren Höhe befinden, als bei bekannten Geräten.

Das Peritonealdialysegerät weist vorzugsweise eine Gesamthöhe von < 1,1 m auf. Seite Breite und Tiefe liegen vorzugsweise im Bereich von < 1 m x < 1 m und vorzugsweise bei < 80 cm x < 80 cm. Der Fuß weist beispielweise eine Breite und Tiefe von < 1 m und vorzugsweise < 80 cm auf und ist vorzugweise ohne Rollen ausgeführt.

Es wird darauf hingewiesen, dass der Begriff "Beutel" stellvertretend für jedes beliebige Behältnis steht, das zur Aufnahme von Lösungen geeignet ist. Diese kann feste oder flexible Wände aufweisen.

Des Weiteren ist der Begriff Schale nicht einschränkend auf eine Schale im engeren Sinne auszulegen, sondern umfasst jede beliebige Aufnahme für den oder die Beutel.

Das Schlauchset umfasst Leitungen und kann ein- oder auch mehrteilig ausgeführt sein.

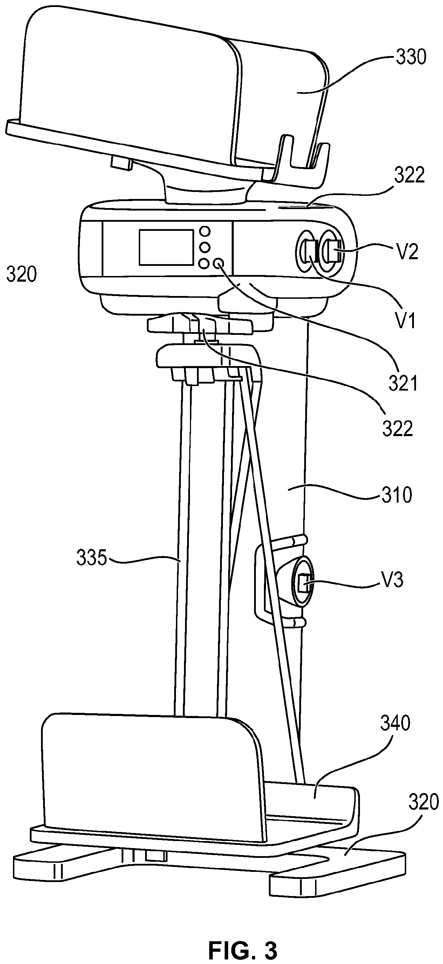

Figur 3 zeigt eine weitere perspektivische Ansicht eines Peritonealdialysegerätes gemäß der Erfindung.

In den Figuren 3 bis 11 sind gleiche oder funktionsgleiche Teile mit denselben Bezugszeichen versehen wie in den Figuren 1 und 2.

Den obersten Punkt des Gerätes bildet die Heizschale 330. Unterhalb der Heizschale befindet sich das Gerätegehäuse 320. In diesem befindet sich ein Einführschlitz 322 z.B. für eine Patientenkarte.

Das Bezugszeichen 321 kennzeichnet das Bedienfeld mit einer oder mehreren Tasten zur Bedienung des Gerätes sowie mit einem Bildschirm, der beispielsweise als herkömmlicher Anzeigebildschirm oder als Touch Screen ausgeführt sein kann.

Das Bezugszeichen V1 kennzeichnet das Ventil, das an dem Gerätgehäuse 320 angeordnet ist und ein Fluidsteuerungsventil bildet, das den Einlauf der Lösung aus den Lösungsbeuteln (außer dem Last-Bag) zum Patienten hin steuert.

Das Bezugszeichen V2 kennzeichnet das Ventil für den Last-Bag, das heißt für den letzten verabreichten Lösungsbeutel.

Wie dies aus Figur 3 hervorgeht, sind beide Ventile am Gerätegehäuse 320 angeordnet.

Mit dem Bezugszeichen 322 ist ein sogenannter Organizer bezeichnet, der zur Aufnahme von Konnektoren des Schlauchsets mit weiteren Leitungen und dem Patientenanschluss dient.

Das Bezugszeichen 310 stellt den Gerätegehäuseträger dar, an dessen oberen Ende sich das Gerätegehäuse 320 und an dessen unteren Ende sich der Gerätefuß 320 befindet.

An dem Gerätegehäuseträger 310 befindet sich das Drainageventil V3, das mit dem Leitungsabschnitt zusammenwirkt, der vom Patienten zu dem oder den Aufnahmebeuteln verläuft sowie mit dem Leitungsabschnitt, der (zu Spülzwecken) von dem oder den Lösungsbeuteln zu dem oder den Aufnahmebeuteln verläuft.

Das Bezugszeichen 335 kennzeichnet ein Gestänge, das an seinem oberen Ende mit einer Wägezelle in Verbindung steht, die in dem Gehäuse 320 angeordnet ist und das an seinem unteren Ende die Wägeschale 340 hält. In dieser befinden sich ein oder mehrere Aufnahmebeutel zur Aufnahme des verbrauchten Dialysats.

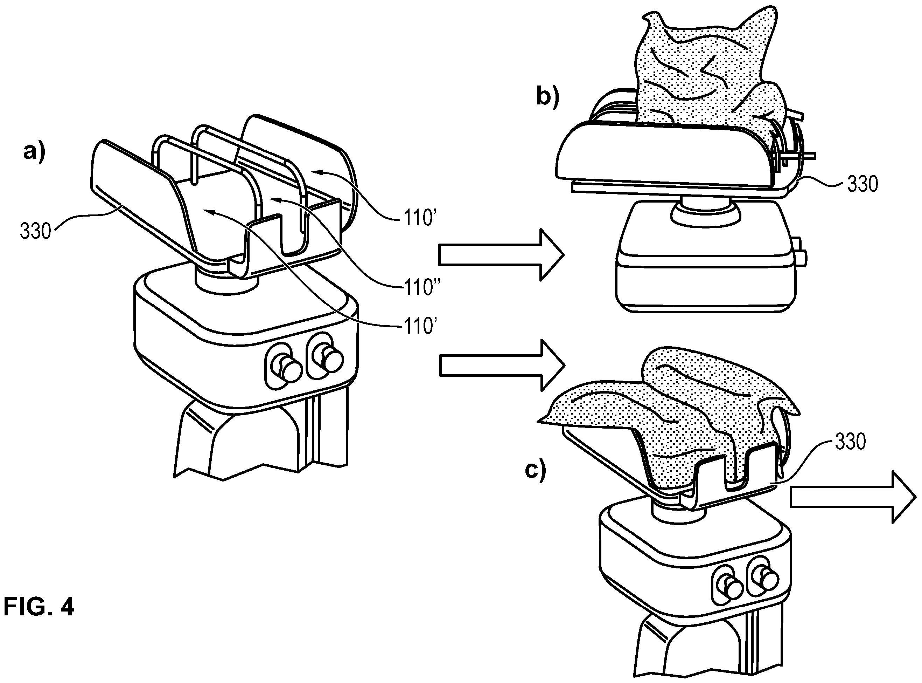

Figur 4a) zeigt in einer perspektivischen Ansicht die Heizschale 330, die durch Bügel oder sonstige Trennelemente in unterschiedliche Bereiche 110', 110" und 110' geteilt ist. Die Heizschale kann auch ohne Trennelemente ausgeführt sein, die deren Aufnahmebereich in mehrere Teilbereiche trennt.

In den Bereichen 110' befinden sich Lösungsbeutel und in dem Bereich 110" vorzugsweise der sogenannte Last-Bag.

Figur 4b) zeigt die Aufnahme eines Einkammerbeutels in der Heizschale 330 und Figur 4c) die Aufnahme eines Doppelkammerbeutels in der Heizschale 330.

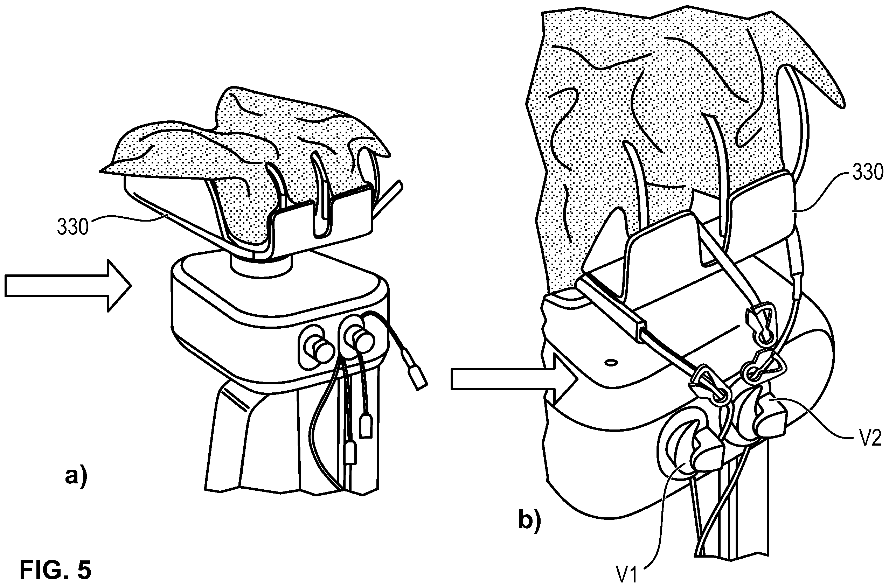

Aus einer besonders bevorzugten Ausführung gemäß Figur 5 ergibt sich der Zustand, in dem die Lösungsbeutel in der Heizschale 330 aufgenommen sind und das Schlauchset bereits in den Ventilen V1 und V2 aufgenommen ist.

Die Lösungsbeutel 100, die nicht den Last-Bag darstellen, sind über ein Y-Stück miteinander verbunden.

Der stromabwärts dieses Y-Stückes verlaufende Leitungsabschnitt befindet sich in dem geräteseitigem Ventil V1. In dem geräteseitig angeordneten Ventil V2 befindet sich die Leitung, die mit dem Last-Bag in Verbindung steht.

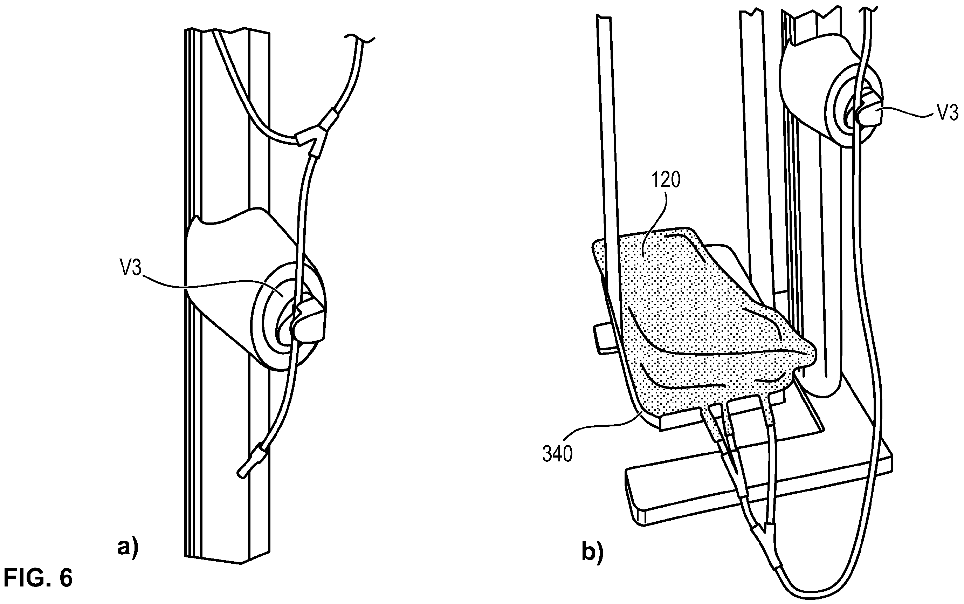

Figur 6 zeigt in vergrößerter Ansicht in der Ansicht a das Drainageventil V3.

Dieses Ventil befindet sich an dem Gerätegehäuseträger 310 und nimmt den Leitungsabschnitt des Schlauchsets auf, der vom Patientenkonnektor und von den Lösungsbeuteln zu dem Aufnahmebeutel bzw. zu einer Leitung führt, die mit den Aufnahmebeuteln 120 in Verbindung steht. Vorzugsweise befinden sich in der in Figur 6b) dargestellten Wägeschale 340 mehrere Aufnahmebeutel 120 zur Aufnahme des gebrauchten Dialysats.

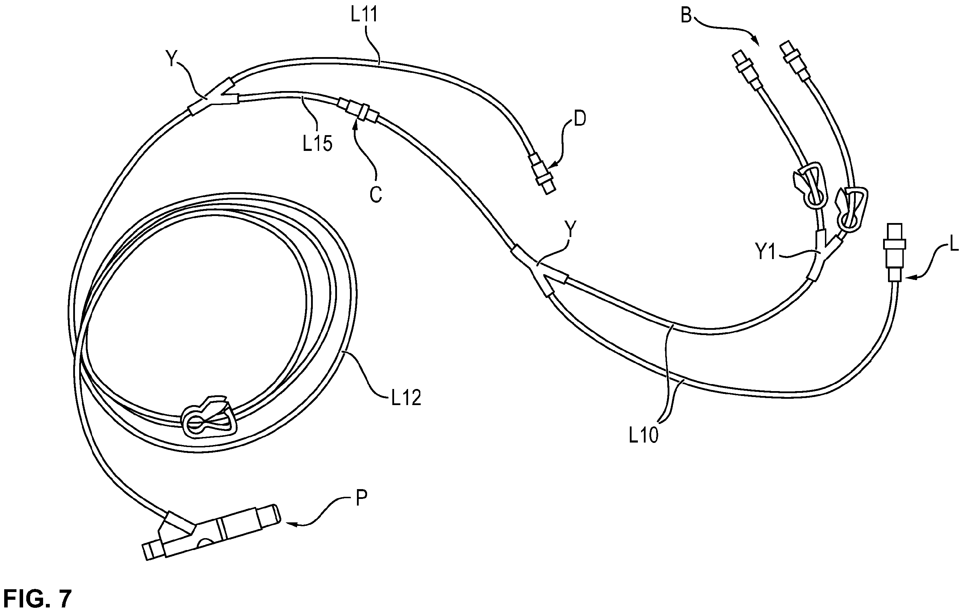

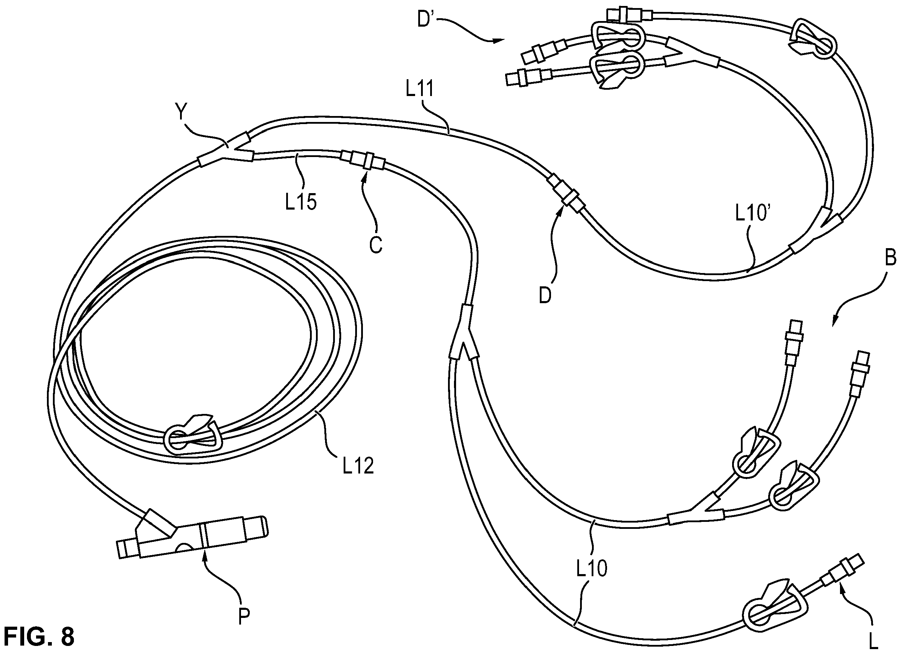

Figur 7 sowie Figur 8 zeigen Schlauchsets, die in einem Peritonealdialysegerät gemäß der vorliegenden Erfindung in bevorzugter Ausgestaltung zur Anwendung kommen.
wobei die ersten Leitungsabschnitte jeweils eine Länge im Bereich von 50 cm bis 80 cm, vorzugsweise im Bereich von 60 cm bis 70 cm, wobei der zweite Leitungsabschnitt eine Länge im Bereich von 25 cm bis 45 cm, vorzugsweise im Bereich von 30 cm bis 40 cm und wobei der dritte Leitungsabschnitt eine Länge im Bereich von 1,9 m bis 2,2 m, vorzugsweise im Bereich von 2,0 m bis 2,1 m aufweisen.

Das Schlauchset umfasst den Patientenkonnektor P und einen von diesem zu einem Y-Stück "Y" führenden Leitungsabschnitt L12. Der Leitungsabschnitt L12 weist vorzugsweise eine Länge im Bereich von 1,9 m bis 2,2 m, vorzugsweise im Bereich von 2,0 m bis 2,1 m auf.

Von dem Y-Stück zweigt eine Leitung L11 ab, deren Ende durch den Konnektor D gebildet wird. An diesem Konnektor wird der Aufnahmebeutel angeschlossen oder eine Leitung, die mit einem oder mehreren Aufnahmebeuteln in Verbindung steht. Der Leitungsabschnitt L11 weist vorzugsweise eine Länge im Bereich von 25 cm bis 45 cm, vorzugsweise im Bereich von 30 cm bis 40 cm auf.

Das Leitungsstück L15 zweigt ebenfalls von dem Y-Stück ab und endet an dem auftrennbaren Konnektor C, bei dem es sich vorzugsweise um einen aufbrechbaren Luer-Lock Konnektor handelt. Der Leitungsabschnitt L15 weist vorzugsweise eine Länge im Bereich von 15 cm bis 25 cm auf.

Von diesem Konnektor C erstreckt sich der Leitungsabschnitt L10 zu den Konnektoren B für die Lösungsbeutel, die nicht den Last-Bag dargestellen und zu dem Konnektor L, der mit dem Last-Bag in Verbindung steht. Die Leitungsabschnitte von dem Y-Stück "Y" bis zu den Konnektoren B für die Lösungsbeutel weisen vorzugsweise eine Länge im Bereich von 50 cm bis 80 cm und vorzugsweise im Bereich von 60 cm bis 70 cm auf. Entsprechendes gilt in bevorzugter Ausgestaltung für den Leitungsabschnitt von dem Y-Stück "Y" bis zu dem Konnektor L für den "Last-Bag". Dieser ist jedoch bevorzugt um 3 cm bis 7 cm länger ausgeführt.

Wie dies aus Figur 7 ersichtlich ist, stehen die Leitungen, die mit den Konnektoren B in Verbindung stehen, über ein Y-Stück Y1 in Verbindung. Die aus diesem mündende gemeinsame Leitung steht über das weitere Y-Stück Y2 mit der Leitung in Verbindung, die zu dem Last-Bag führt.

Die Länge des Leitungsabschnittes zwischen dem Konnektor C und dem Y-Stück "Y2" liegt vorzugweise im Bereich von 3 cm bis 7 cm, die Länge des Leitungsabschnittes zwischen dem Y-Stück "Y2" und dem Y-Stück "Y1" liegt vorzugsweise im Bereich von 15 cm bis 25 cm und, die Länge des Leitungsabschnittes zwischen dem Y-Stück "Y2" und dem Konnektor L liegt vorzugsweise im Bereich zwischen 40 cm und 50 cm und die Länge des Leitungsabschnittes zwischen dem Y-Stück "Y1" und den Konnektoren B liegt vorzugsweise im Bereich zwischen 15 cm bis 25 cm.

Aus Figur 8 ist eine Anordnung ersichtlich, die im Wesentlichen der zu Figur 7 erläuterten entspricht, sodass entsprechend Bezug genommen wird. In der Anordnung gemäß Figur 8 ist das Leitungssystem L10 an dem Konnektor C aufgetrennt worden und mit einem neuen Schlauchset verbunden worden. Die bisherigen Leitungen L10 des benutzten Schlauchsets sind in Figur 8 mit dem Bezugszeichen L10` gekennzeichnet. Sie werden an den Konnektor D eines neuen Schlauchsets angeschlossen und stehen mit ihren Endbereichen mit Aufnahmebeuteln in Verbindung, die in der Wägeschale 340 aufgenommen sind.

Somit werden jeweils Leitungsabschnitte L10, die mit den Lösungsbeuteln 100 in Verbindung stehen nach der Behandlung bzw. nach dem Einlauf in den Patienten an der Stelle C abgetrennt und mit dem Konnektor D eines neuen Schlauchsets verbunden.

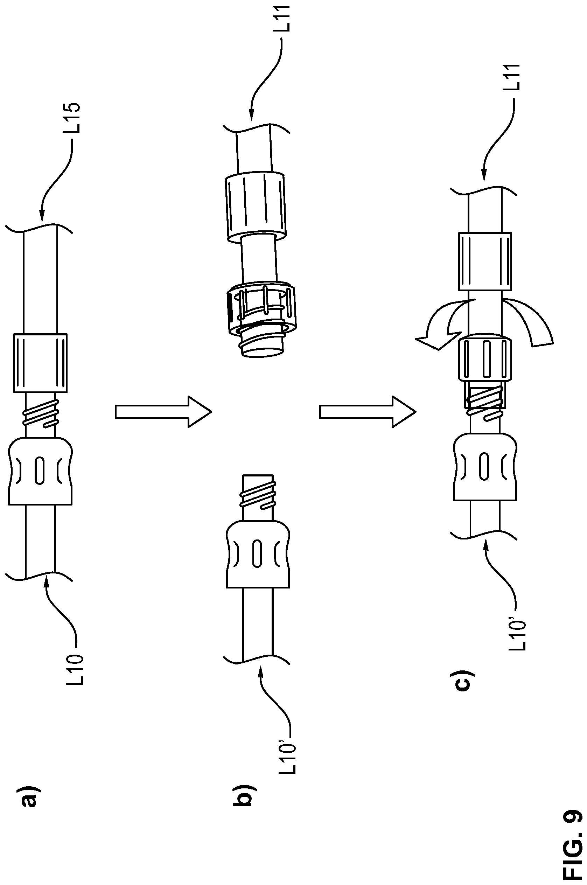

Dieser Vorgang ergibt sich auch aus Figur 9. Figur 9a zeigt mit dem Bezugszeichen L10 die Leitung, die zu den entleerten Lösungsbeuteln 100 führt und mit dem Bezugszeichen L15 die über den Konnektor mit dieser Leitung in Verbindung stehende Leitung des Schlauchsets. Diese Leitungsverbindung wird aufgetrennt und das Leitungssystem L10` gemäß Figur 8 nun mit einem neuen Schlauchset, das in Figur 9b mit dem Bezugszeichen L11 gekennzeichnet ist, verbunden. Den Vorgang der Verbindung lässt sich aus Figur 9c entnehmen.

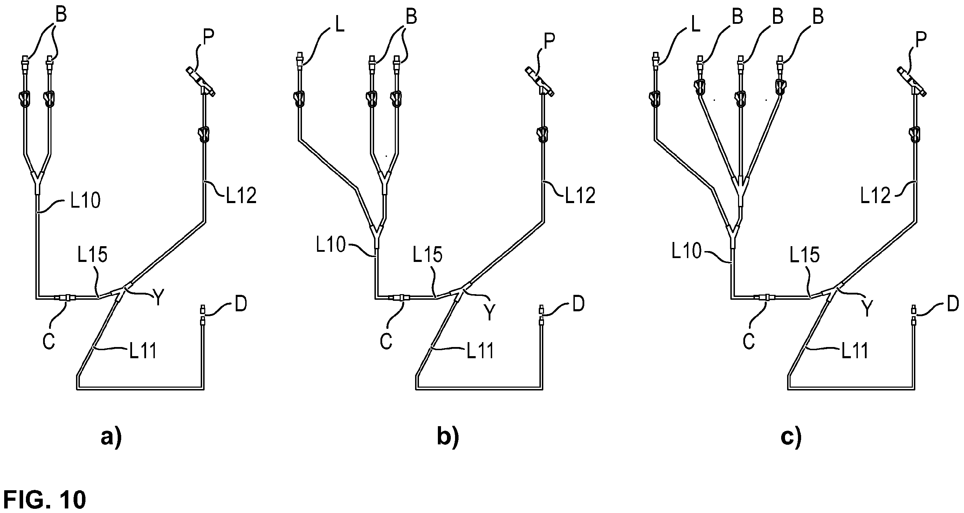

Figur 10 zeigt unterschiedliche Ausführungsformen von Schlauchsets gemäß der Erfindung, wobei gleiche Bezugszeichen auf gleiche oder funktionsgleiche Elemente verweisen, sodass entsprechend Bezug genommen wird.

Figur 10a zeigt ein Schlauchset ohne eine Leitung für den Last-Bag sondern mit zwei Leitungsanschlüssen bzw. Konnektoren B für Lösungsbeutel.

Figur 10b) zeigt eine Anordnung, bei der zusätzlich ein Last-Bag verwendet wird, der über den Konnektor L verbunden wird und Figur 10c) zeigt eine Anordnung mit drei Leitungen bzw. Konnektoren B für Lösungsbeutel und einem Anschluss L für den Last-Bag.

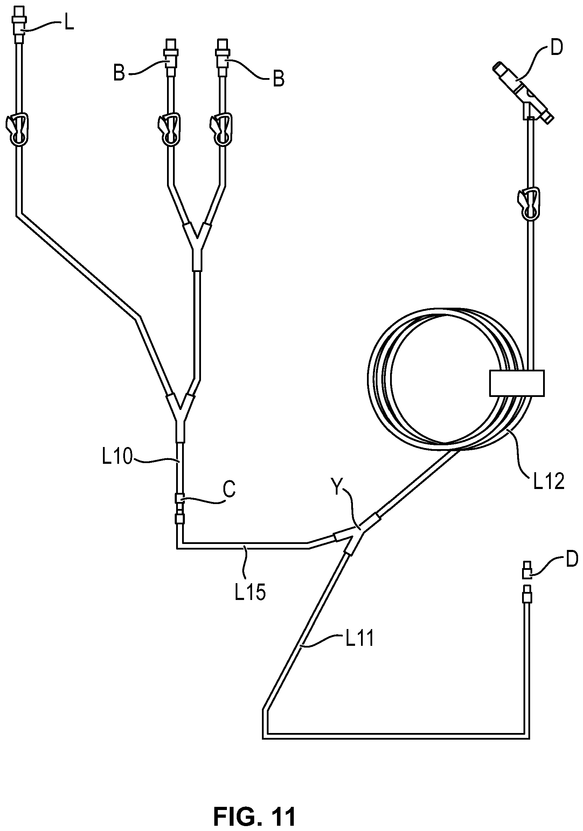

Die Anordnung gemäß Figur 10b) ist nochmals in Figur 11 wiedergegeben.

Aus dieser Figur ergibt sich auch die lösbare Verbindungsstelle C, an der nach dem Einlaufen der Lösungen in den Patienten die Leitung L10 von der Leitung L15 getrennt wird. Diese Leitung L10 wird dann als Leitung L10' an den Konnektor D eines neuen Schlauchsets angesetzt und mit Aufnahmebeuteln verbunden.

## Patentansprüche

1. Schlauchset für ein Peritonealdialysegerät, **dadurch gekennzeichnet, dass** das Schlauchset erste Leitungsabschnitte (L10) aufweist, die sich von den Anschlüssen (B) für die Lösungsbeutel zu einem Verzweigungsstück (Y) erstrecken, wobei mit dem Verzweigungsstück (Y) ein zweiter Leitungsabschnitt (L11) in Verbindung steht, der sich von dem Verzweigungsstück (Y) zu dem Anschluss (D) für den oder die Aufnahmebeutel oder eine oder mehrere damit in Verbindung stehende Leitungen erstreckt, und wobei mit dem Verzweigungsstück (Y) ein dritter Leitungsabschnitt (L12) in Verbindung steht, der sich von dem Verzweigungsstück (Y) zu dem Anschluss für die Patientenleitung erstreckt.

2. Schlauchset gemäß Anspruch 1, wobei die ersten Leitungsabschnitte jeweils eine Länge im Bereich von 50 cm bis 80 cm, vorzugsweise im Bereich von 60 cm bis 70 cm, wobei der zweite Leitungsabschnitt eine Länge im Bereich von 25 cm bis 45 cm, vorzugsweise im Bereich von 30 cm bis 40 cm und wobei der dritte Leitungsabschnitt eine Länge im Bereich von 1,9 m bis 2,2 m, vorzugsweise im Bereich von 2,0 m bis 2,1 m aufweisen.

3. Schlauchset gemäß Anspruch 1 oder 2, wobei weiterhin von dem Y-Stück eine Leitung (L15) abzweigt, welche an einem auftrennbaren Konnektor (C) endet, von dem sich die ersten Leitungsabschnitte (L10) zu den Konnektoren (B) für Lösungsbeutel erstrecken, wobei die ersten Leitungsabschnitte (L10) an dem Konnektor (C) abtrennbar sind und nach einem Einlauf der Lösung aus den Lösungsbeuteln in einen Patienten mit dem Konnektor (D) eines neuen Schlauchsets verbindbar oder verbunden sind.

4. Schlauchset nach einem der vorhergehenden Ansprüche, **dadurch gekennzeichnet, dass** der Konnektor (C) ein aufbrechbarer Luer-Lock-Konnektor ist.

5. Schlauchset nach einem der vorhergehenden Ansprüche, **dadurch gekennzeichnet, dass** der Konnektor (C) und / oder der Konnektor (D) eine lösbare Schraubverbindung aufweist.

6. Schlauchset nach einem der vorhergehenden Ansprüche, **dadurch gekennzeichnet, dass** das Schlauchset derart ausgebildet ist, dass es zeitgleich mit zwei oder mehr als zwei Lösungsbeuteln unterschiedlichen Inhalts verbunden ist, so dass eine Mischlösung hergestellt werden kann.

7. Schlauchset nach einem der vorhergehenden Ansprüche, **dadurch gekennzeichnet, dass** das Schlauchset derart an einem Peritonealdialysegerät angeordnet ist, dass das Schlauchset zwischen dem oder den Anschlüssen für einen oder mehrere Lösungsbeutel und dem Ende des Schlauchsets, mit dem dieses mit der Patientenleitung verbunden wird, nur mit genau einem Ventil (Fluidsteuerungsventil) des Peritonealdialysegerätes zusammenwirkt, und/oder dass das Schlauchset derart angeordnet ist, dass das Schlauchset zwischen dem Ende des Schlauchsets, mit dem dieses mit der Patientenleitung in Verbindung steht und dem Anschluss für den Aufnahmebeutel nur mit genau einem Ventil (Drainageventil) des Peritonealdialysegerätes zusammenwirkt.

8. Schlauchset nach einem der vorhergehenden Ansprüche, **dadurch gekennzeichnet, dass** das Schlauchset als Disposable ausgebildet ist und/oder dass das Schlauchset zwei, drei oder vier oder mehr als vier Anschlüsse für die Lösungsbeutel, einen Anschluss für den oder die Aufnahmebeutel und einen Anschluss für die Verbindung mit der Patientenleitung aufweist.

9. Schlauchset nach einem der vorhergehenden Ansprüche, **dadurch gekennzeichnet, dass** das Schlauchset eine Gesamtlänge von 3 m bis 4,5 m, vorzugsweise im Bereich von 3,5 m bis 4,0 m aufweist.

10. Verwendung eines Schlauchsets gemäß einem der vorhergehenden Ansprüche mit einem Peritonealdialysegerät, wobei das Schlauchset nicht mit einem Heizbeutel in Verbindung steht, in den die aus den Lösungsbeuteln ablaufende Flüssigkeit einläuft und/oder wobei das Schlauchset nicht mit einem Wägebeutel in Verbindung steht, aus dem das gebrauchte Dialysat in einen Abfallbeutel einläuft.

11. Verwendung gemäß Anspruch 10 , wobei nach einem Einlauf der Lösung aus den Lösungsbeuteln in einen Patienten die ersten Leitungsabschnitte (L10) an dem Konnektor (C) abgetrennt werden und mit dem Konnektor (D) eines neuen Schlauchsets verbunden werden.
